# EUROPEAN PATENT SPECIFICATION

(11) **EP 3 648 680 B1**
(45) Date of publication and mention of the grant of the patent: **10.03.2021**
(21) Application number: 18746404.5
(22) Date of filing: 06.07.2018
(51) Int. Cl.: A61B 17/04, A61B 17/06, A61B 17/00

(54) **SUTURE-IMPLANT CONSTRUCTS AND SURGICAL DEVICE FOR DELIVERING SAME**
SUTURE-IMPLANTAT-KONSTRUKTE UND CHIRURGISCHE VORRICHTUNG ZUR ABGABE DERSELBEN
STRUCTURES D'IMPLANT DE SUTURE ET DISPOSITIF CHIRURGICAL POUR LES METTRE EN PLACE

(30) Priority: 07.07.2017 US 201715643520; 08.01.2018 US 201815864948
(43) Date of publication of application: 13.05.2020
(73) Proprietor: Arthrex, Inc., Naples, FL 34108-1945 (US)
(72) Inventor: BEST, Joshua J., Naples, Florida 34116 (US); HELENBOLT, Kenneth T., Naples, Florida 34109 (US); OSIKA, Andrew, Naples, Florida 34119 (US); HAUGHTON, Timothy J., Newton, Massachusetts 12462 (US); DEAN, Marshall, Wakefield, Massachusetts 01880 (US); BERMUDEZ, Omar, Brookline, Massachusetts 02445 (US); SEARS, Charles W., Boxford, Massachusetts 01921 (US); JOHNSON, Timothy N., Freeport, Maine 04032 (US)
(74) Representative: Lohr, Jöstingmeier & Partner
(86) International application number: PCT/US2018/041032
(87) International publication number: WO 2019/010380

(56) References cited:
- WO-A1-2016/068896
- WO-A2-2006/086275
- US-A1- 2008 140 092
- US-A1- 2008 140 093

## Description

### BACKGROUND

This disclosure relates to a suture-implant construct, a surgical device configured to deliver the suture-implant construct, and a method for repairing damaged tissue.

Orthopedic procedures are often performed to repair musculoskeletal injuries, such as those sustained during sporting activities. Tears in the meniscus are known to be repaired by deploying implants on either side of the tear, cinching suture between the implants to close the tear, and allowing it to heal. A surgical device for soft tissue repair is disclosed in US 2008/0140093.

### SUMMARY

The invention is defined in claim 1. Preferred embodiments of the invention are recited in the dependent claims. This disclosure relates to a surgical device configured to deliver implants and a method of repairing damaged tissue, such as meniscus tears. One exemplary surgical device includes a cannula and a pusher moveable within the cannula to deploy a plurality of implants. Movement of a pusher deploys a distal-most implant and moves any additional implants distally within the cannula. Thus, multiple implants can be loaded into the cannula and deployed using one pusher. The disclosed arrangement is easy to use and has fewer component parts compared to prior devices, which in turn increases the ease of manufacture and reduces cost.

A surgical device according to an exemplary aspect of the present disclosure includes, *inter alia,* a cannula and a suture-implant construct within the cannula. The suture-implant construct includes a strand of suture and at least two implants. The first implant is in a deploy position, and the remaining implant(s) is in a standby position. For example, a surgical device includes a pusher moveable within the cannula to deploy a first implant and to move a second implant from the standby position to the deploy position.

A method according to an exemplary aspect of the present disclosure includes, *inter alia,* moving a pusher of a surgical device to deploy a first implant of a suture-implant construct out of a cannula and to move a second implant of the suture-implant construct distally within the cannula. The method also includes moving the pusher in a proximal direction, and moving the pusher in the distal direction again to deploy the second implant out of the cannula.

This disclosure relates to suture-implant constructs including one or more sheaths and a flexible strand, such as a strand of suture, suture tape, and the like. The disclosure also relates to surgical devices configured to deliver the suture-implant constructs and methods of repairing damaged tissue. An exemplary suture-implant construct includes a strand of suture and one or more sheaths arranged to provide a plurality of spaced-apart anchor portions. When used to repair a tear in a meniscus, for example, the disclosed arrangement resists "tear-through".

A suture-implant construct includes, *inter alia,* a strand of suture and one or more sheaths and each sheath comprising a plurality of spaced-apart anchor portions.

A surgical device includes, *inter alia,* a cannula, and a suture-implant construct comprising a strand of suture and one or more sheaths, and each sheath comprising a plurality of anchor portions. A first anchor portion can be in a deploy position with respect to the cannula and a second anchor portion can be in a standby position proximal to the first anchor portion. A surgical device can further include a pusher moveable within the cannula to deploy the first anchor portion and to move the second anchor from the standby position to a deploy position.

A method includes, *inter alia,* moving a pusher of a surgical device in a distal direction to deploy a first anchor portion of a suture-implant construct out of a cannula and to move a second anchor portion of the suture-implant construct distally within the cannula. The suture-implant construct has a strand of suture and one or more sheaths, and each sheath comprising a plurality of anchor portions. A method can further include moving the pusher in a proximal direction and subsequently moving the pusher in the distal direction again to deploy the second anchor portion out of the cannula.

### BRIEF DESCRIPTION OF THE DRAWINGS

Figure 1 illustrates an example surgical device held by a user, such as a surgeon.
Figures 2A and 2B are perspective views of the surgical device of Figure 1 with a suture-implant construct and a pusher partially removed from the surgical device for purposes of illustration, according to exemplary embodiments, respectively.
Figure 3 is a top view of a distal end of the surgical device, and illustrates the arrangement between a cannula and the pusher.
Figure 4 is a side view illustrating a shuttling rack.
Figure 5 is a side view illustrating the distal end of the pusher.
Figure 6 is a cross-sectional view illustrating the surgical device with the entire suture-implant construct loaded into the cannula.
Figure 7 is a view of the surgical device penetrating a meniscus in a first location adjacent a meniscus tear.
Figure 8 is a cross-sectional view illustrating the surgical device with a first implant deployed.
Figure 9 is a view of the surgical device as the first implant is deployed in the first location adjacent the meniscus tear.
Figure 10 is a cross-sectional view illustrating the surgical device with a second implant moved to a deploy position.
Figures 11A and 11B are views of the surgical device penetrating a meniscus in a second location adjacent the meniscus tear, according to exemplary embodiments, respectively.
Figure 12 is a cross-sectional view illustrating the surgical device with the second implant deployed.
Figures 13A and 13B are views of the surgical device as the second implant is deployed in the second location adjacent the meniscus tear, according to exemplary embodiments, respectively.
Figure 14 is a top view of a suture-implant construct with the second anchor portion deployed in the second location.
Figures 15A and 15B are views of a closed meniscus tear according to the exemplary embodiments.
Figure 16 is a top view of another suture-implant construct with the second anchor portion deployed in the second location.
Figure 17 is a perspective view of another surgical device loaded with a suture-implant construct that has more than one sheath.

### DETAILED DESCRIPTION

This disclosure relates to a surgical device configured to deliver implants and a method of repairing damaged tissue, such as meniscus tears. An exemplary surgical device includes a cannula and a pusher moveable within the cannula to deploy a plurality of implants. Movement of the pusher deploys a distal-most implant and moves any additional implants distally within the cannula. Thus, multiple implants can be loaded into the cannula and deployed using one pusher. The disclosed arrangement is easy to use and has fewer component parts compared to prior devices, which in turn increases the ease of manufacture and reduces cost.

A surgical device according to an exemplary aspect of the present disclosure includes, *inter alia,* a cannula and a suture-implant construct including a strand of suture and at least two implants. The first implant is in a deploy position and the second implant is in a standby position proximal to the first implant. The surgical device further includes a pusher moveable within the cannula in a distal direction to deploy the first implant and to move the second implant from the standby position to the deploy position.

In a further embodiment of the foregoing surgical device, a pusher includes a shuttling rack in contact with the second implant when the second implant is in the standby position.

In a further embodiment of any of the foregoing surgical devices, the shuttling rack is integrated into the pusher.

In a further embodiment of any of the foregoing surgical devices, the cannula includes a slot, and the pusher is arranged within the slot.

In a further embodiment, a pusher includes an elongate shaft extending along a cannula.

In a further embodiment, a shuttling rack includes a plurality of barbs on a side of the elongate shaft.

In a further embodiment, each of the plurality of barbs has a distal face and proximal face meeting at an apex. Further, the distal face is substantially normal to a longitudinal axis of the cannula, and the proximal face is inclined at an acute angle relative to the distal faces.

In a further embodiment, a surgical device further includes a trigger configured to move in a distal direction and a proximal direction, and the trigger is mechanically coupled to the pusher such that movement of the trigger moves the pusher.

In a further embodiment, a trigger is a thumb trigger.

In a further embodiment, a trigger is biased in the proximal direction.

In a further embodiment, a surgical device includes a brake configured to be selectively activated to overcome a bias of a trigger and maintain a position of the trigger and a pusher.

In a further embodiment, implants are tubular sleeves made of a flexible material.

In a further embodiment, implants are made of a polyester suture material.

In a further embodiment, a suture-implant construct includes a third implant proximal to the second implant and a fourth implant proximal to the third implant.

A method according to an exemplary aspect of the present disclosure includes, *inter alia,* moving a pusher of a surgical device in a distal direction to deploy a first implant of a suture-implant construct out of a cannula and to move a second implant of the suture-implant construct distally within the cannula. The method also includes moving the pusher in a proximal direction, and moving the pusher in the distal direction again to deploy the second implant out of the cannula.

In a further embodiment, a method includes first and second implants that are implanted into a meniscus to repair a tear in the meniscus.

In a further embodiment, a method includes the pusher comprising a shuttling rack having barbs inclined such that the shuttling rack only moves the second implant in the distal direction.

In a further embodiment, a method includes moving a pusher in a distal direction to apply an input force to a trigger to overcome bias in the proximal direction.

In a further embodiment, a method includes braking a pusher to hold the position of the pusher after moving the pusher in the distal direction and before the pusher moves back to the proximal direction under the bias.

This disclosure also relates to suture-implant constructs including one or more sheaths and a flexible strand, such as a strand of suture, suture tape, or the like. The disclosure also relates to surgical devices configured to deliver the suture-implant constructs as disclosed herein and methods of repairing damaged tissue. An exemplary suture-implant construct includes a strand of suture and one or more sheaths, and each sheath comprises at least a first anchor portion and a second anchor portion spaced-apart from the first anchor portion. When used to repair a tear in a meniscus, for example, the disclosed arrangement resists "tear-through".

A suture-implant construct includes, *inter alia,* a strand of suture and one or more sheaths each arranged to provide a plurality of spaced-apart anchor portions.

In a further embodiment, each sheath has a first end and a second end, the first anchor portion is adjacent the first end of the sheath, and the second anchor portion is adjacent the second end of the sheath.

In a further embodiment, the strand of suture passes through a bore of the sheath between the first and second ends thereof.

In a further embodiment, each sheath has an increased width dimension between the first and second anchor portions.

In a further embodiment, the first anchor portion includes at least one splice point in which the strand of suture exits and re-enters the bore, and the second anchor portion includes at least one splice point in which the strand of suture exits and re-enters the bore.

In a further embodiment, the first anchor portion includes two splice points spaced-apart from one another, and the second anchor portion includes two splice points spaced-apart from one another.

In a further embodiment, an end of the strand of suture is looped-over and affixed to the strand of suture adjacent one of the two splice points of the first anchor portion.

In a further embodiment, the strand of suture includes a bulb adjacent the first end of the sheath.

In a further embodiment, the strand of suture is a monofilament suture including barbs.

In a further embodiment, the sheath is a tubular sleeve made of a flexible material, such as polyester suture material or the like.

A surgical device includes, *inter alia,* a cannula, and a suture-implant construct comprising a strand of suture and at least one sheath comprising at least a first anchor portion and a second anchor portion. The first anchor portion can be in a deploy position with respect to the cannula and the second anchor portion can be in a standby position proximal to the first anchor portion. In an embodiment, third, fourth, fifth, etc. anchor portions of the sheath can be in a standby position. A surgical device can further include a pusher moveable within the cannula to deploy the first anchor portion and to move any subsequent anchor portions from the standby position to the deploy position.

In a further embodiment, the pusher includes a shuttling rack in contact with the second anchor portion of the sheath when the second anchor portion is in the standby position. Where the shuttling rack can be tubular or rectangular. In one embodiment, the shuttling rack includes a plurality of barbs.

In a further embodiment, the pusher is a holder tube that extends through and moves freely with respect to the sheath.

In a further embodiment, the sheath has a first end and a second end, the first anchor portion is adjacent the first end of the sheath, the second or subsequent anchor portion is adjacent to the second end of the sheath, the strand of suture passes through a bore of the sheath between the first and second ends, and the sheath has an increased width dimension between the plurality of anchor portions.

A method according to an exemplary aspect of the present disclosure includes, *inter alia,* moving a pusher of a surgical device in a distal direction to deploy a first anchor portion of a suture-implant construct out of a cannula and to move at least a second anchor portion of the suture-implant construct distally within the cannula. The suture-implant construct can have a strand of suture and one or more sheaths arranged to provide a plurality of anchor portions. A method can further include moving a pusher in a proximal direction and subsequently moving the pusher in the distal direction again to deploy the second anchor portion out of the cannula.

In a further embodiment, the anchor portions are implanted into a meniscus to repair a tear in the meniscus.

In a further embodiment, a method includes tensioning the strand of suture to close the tissue repair, such as a meniscal tear.

In a further embodiment, the anchor portions are implanted through a surface of the torn meniscus to outside the knee capsule. The sheath can also cover the strand of suture between the anchor portions and extend along the surface of the meniscus. In a further embodiment, the sheath has an increased width dimension on the surface of the meniscus.

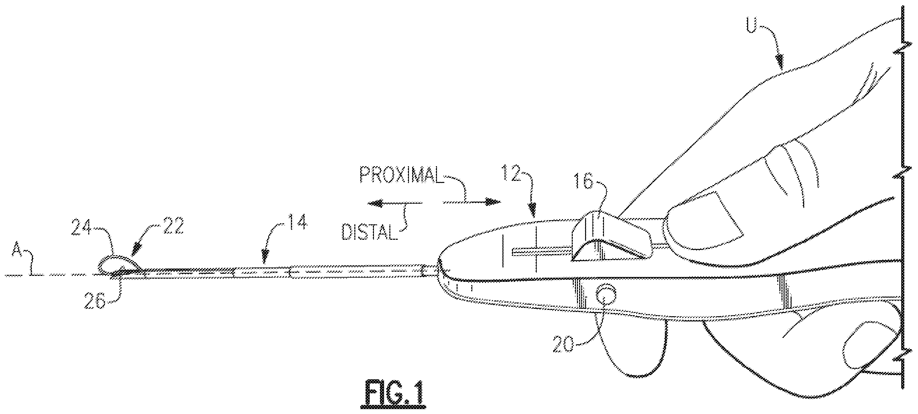

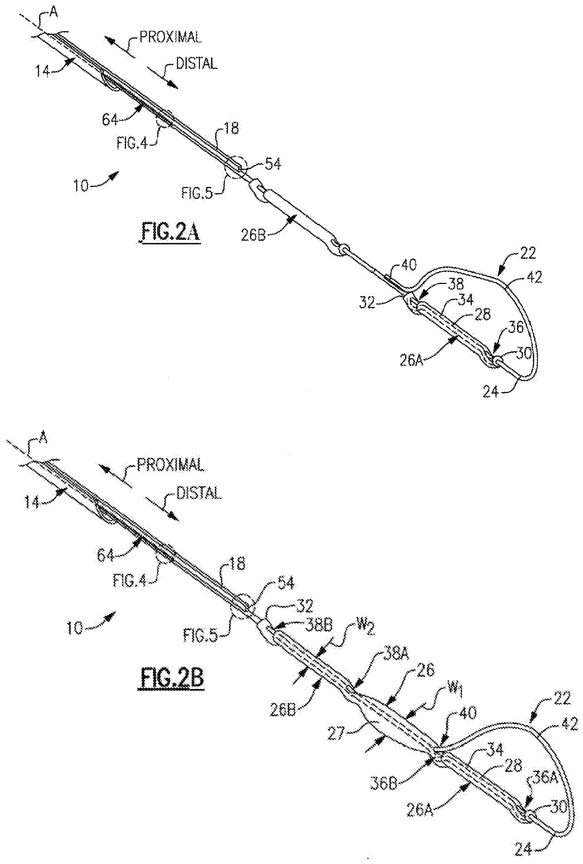

Figure 1 illustrates an example surgical device 10. The surgical device 10 includes a handle 12 and a cannula 14 projecting distally (the "distal" direction is labeled in various figures for reference) from the handle 12 along a longitudinal axis A. The handle 12 includes a trigger 16, which in this example is a thumb trigger. The trigger 16 is moveable in the distal and proximal directions (the "proximal" direction is labeled in various figures reference) to move a pusher 18 (Figure 2), which itself is moveable within the cannula 14 in the distal and proximal directions.

In one example, the handle 12 includes a spring or other biasing element configured to bias the trigger 16 in the proximal direction. In order to move the trigger 16 in the distal direction, a user U (i.e., a surgeon) uses their thumb, for example, to apply a force to the trigger 16 sufficient to overcome the bias of the spring such that the trigger 16 slides distally. When the user U releases their thumb, the trigger 16 moves proximally back to a resting position under the bias of the spring or other biasing element.

The handle 12 may optionally incorporate a brake. In this example, the brake is selectively activated by depressing a button 20 disposed on an exterior surface of the handle 12. When the button 20 is depressed, the brake engages the trigger 16, or a structure associated with the trigger 16, to hold the trigger 16 in place and overcome the proximal bias of the trigger 16. This braking function is useful in some circumstances, such as when penetrating the meniscus with the cannula 14, for example.

The cannula 14 is configured to penetrate soft tissue within the body. To this end, a distal-most tip of the cannula 14 is relatively sharp and may be tapered or pointed in some examples. Further, the cannula 14 is loaded with a suture-implant construct 22 (sometimes referred to as a "suture construct"), which includes a strand of suture 24 and at least one implant 26. A user can position the cannula 14 adjacent a meniscus tear, for example, and use the trigger 16 to selectively deploy the at least one implant 26 or the suture-implant construct 22.

Figures 2A and 2B illustrate a distal end of the surgical device 10 with the pusher 18 and suture-implant construct 22 partially removed from the cannula 14 for ease of reference. In this example, the suture-implant construct 22 includes a first implant 26A and a second implant 26B, each of which are arranged along the strand of suture 24. The first implant 26A is distal of the second implant 26B. As shown, the sheath 26 includes a distal end 30 and a proximal end 32. The first anchor portion 26A is adjacent the distal end and is distal of the second anchor portion 26B. The second anchor portion 26B is adjacent the proximal end 32 and is proximal of the first anchor portion 26A. In an embodiment, the sheath 26 and its anchor portions 26A and 26B are one-piece.

It should be understood that while two anchor portions 26A, 26B are illustrated, the sheath may include additional anchor portions. In one particular example, the strand of suture 24 and/or the strand 24 and the sheath 26 are arranged to provide the suture-implant construct with four anchor portions arranged sequentially along the strand of suture 24. In an embodiment, the four anchor portions are space-apart from one another.

The sheath 26 has a substantially tubular body that extends between the distal and proximal ends 30, 32. In this example, the sheath 26 is provided by a single, integrated body. The sheath 26 further includes a bore 34 (illustrated in phantom) communicating with the distal and proximal ends 30, 32 for accommodating the strand of suture 24. The strand of suture 24 is flexible and is passed through the bore 34. In the illustrated examples, the strand of suture 24 is encased by the sheath 26 throughout substantially the entire length of the sheath 26, with the exception of a plurality of splice points.

Each of the first and second anchor portions 26A, 26B includes at least one splice point, in which the strand of suture 24 exits and re-enters the bore 34. In this example, the first anchor portion 26A includes two splice points 36A, 36B, and the second anchor portion 26B includes two splice points 38A, 38B. The strand of suture 24 exits and re-enters the bore 34 through each of the splice points 36A, 36B, 38A, 38B. The distal-most and proximal-most splice points 36A, 38B are spaced inward of respective ends 30, 32 of the sheath 26. When the first and second anchor portions 26A, 26B are deployed adjacent soft tissue, tension is applied to the strand of suture 24, which cinches the first and second anchor portions 26A, 26B, anchoring them in place.

As seen in Figure 2B, the sheath 26 may include a central portion 27, which spans the entirety of the distance between the first and second anchor portions 26A, 26B. The sheath 26 covers the strand of suture 24 throughout the central portion 27. That is, there are no splice points between the first and second anchor portions 26A, 26B. As will be appreciated from the below discussion, the central portion 27 of the sheath 26 may be positioned on a surface of torn tissue, such as on the medial or inside surface of a meniscus. Therefore, this central portion 27 of the sheath 26 may be positioned adjacent the condyles of the knee, for example. In this way, the sheath 26 serves to protect the condyles of the knee from abrasion that may be caused by the strand of suture 24, which may include barbs.

Further, the sheath 26 also serves to prevent "tear through," which is when an implant tears through soft tissue. In the illustrated embodiment, the central portion 27 of the sheath 26 includes an increased width dimension W₁ relative to the remainder of the sheath 26, such as at locations adjacent the first or second anchor portions 26A, 26B, which have a relatively reduced width dimension W₂. As seen in Figure 2B, the sheath 26 is substantially circular in cross-section adjacent the first and second anchor portions 26A, 26B and is relatively flat or ovular in cross-section adjacent the central portion 27. The increased width dimension W₁ disperses the force applied by the strand of suture 24 over a relatively large surface area of soft tissue, which reduces the risk of "tear through."

As seen in Figure 2A, the first implant 26A includes a sheath 28 having a tubular body that extends between opposing ends 30, 32 of the sheath 28. The sheath 28 further includes a bore 34 communicating with the opposing ends 30, 32 for accommodating the strand of suture 24. In an embodiment, the sheath 28 is a tubular sleeve made of a flexible material, such as a braided, woven, or knitted structure made of yarns, fibers, filaments, sutures, or other similar materials, or combinations of these materials. In another embodiment, the sheath 28 is constructed of polyester suture material. Other flexible materials may also be suitable for constructing the sheath 28.

The strand of suture 24 is flexible and is passed through the bore 34 of the sheath 28. The strand of suture 24 also exits and re-enters the bore 34 through a plurality of splice points 36, 38 of the sheath 28. The splice points 36, 38 are spaced from the ends 30, 32 of the sheath 28. When the first implant 26A is deployed adjacent soft tissue, tension is applied to the suture 24, which cinches the first implant 26A, anchoring it in place. While the above discussion focuses on the detail of the first implant 26A, it should be understood that the second implant 26B is arranged in the same manner.

In this embodiment, an end 40 of the strand of suture 24 is looped over the distal-most implant of the suture-implant construct 22, which in this example is the first implant 26A. In particular, the end 40 is passed through the first implant 26A, and is bent back in the proximal direction and affixed to the strand of suture 24 at a location proximal to the first implant 26A (Figure 2A). The end 40 may be affixed back to the strand of suture 24 by a knot or using some other fixation technique. The result is a loop 42 enclosing a portion of the first implant 26A. The loop 42 retains the first implant 26A relative to the stand of suture 24 and ensures that the first implant 26A does not slide distally off the strand of suture 24.

As seen in Figure 2B, the end 40 of the strand of suture 24 is looped over and affixed to the strand of suture 24 adjacent one of the two splice points 36A, 36B of the first anchor portion 26A. In this example, the end 40 is passed through the first anchor portion 26A, and is bent back in the proximal direction and affixed to the strand of suture 24 at a location adjacent the splice point 36B. The end 40 may be affixed back to the strand of suture 24 by a knot or using some other fixation technique. The result is a loop 42 enclosing a portion of the first anchor portion 26A. The loop 42 retains the sheath 26 relative to the stand of suture 24 and ensures that the sheath 26 does not slide distally off the strand of suture 24.

The suture-implant construct 22 may be referred to as a "soft" construct because it is formed of soft materials such as yarns, fibers, filaments, strings, fibrils, strands, sutures, etc., or any combination of such materials. The soft materials may be synthetic or natural materials, or combinations of synthetic and natural materials, and may be biodegradable or non-biodegradable within the scope of this disclosure. In an embodiment, the suture-implant construct 22 is made exclusively of soft, suture-based materials. The soft materials confer the ability to be inserted into or through tissue (e.g., bone, ligament, tendon, cartilage, etc.) and then bunch together, collapse, expand, and/or change shape to fixate the suture-implant construct 22 relative to the tissue.

In an embodiment, the sheath 26 is a tubular sleeve made of a flexible material, such as a braided, woven, or knitted structure made of yarns, fibers, filaments, sutures, or other similar materials, or combinations of these materials. In another embodiment, the sheath 26 is constructed of polyester suture material. Other flexible materials may also be suitable for constructing the sheath 26.

In an embodiment, the strand of suture 24 is provided by one of the following example types of suture: FiberWire®, TigerWire®, or FiberChain® suture, which are each available from Arthrex, Inc. It should be understood, however, that any type of suture may be used, including cored or coreless sutures. In another embodiment, the strand of suture 24 is flat suture, such as FiberTape® or SutureTape® suture, which are also available from Arthrex, Inc. The strand of suture 24 may also be a monofilament suture. Further, the strand of suture 24 could include any soft, flexible strand of material, and is not limited to suture. The strand of suture 24 may also be a monofilament suture having barbs, as mentioned above.

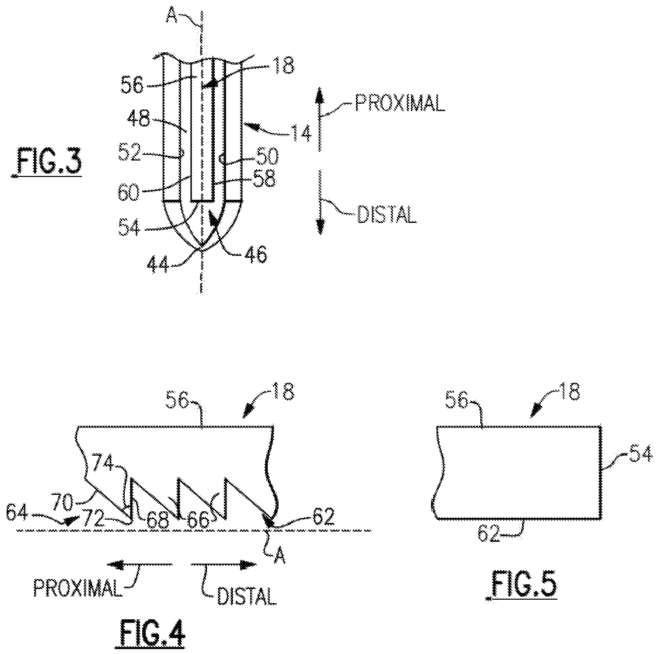

Figure 3 illustrates the arrangement between the cannula 14 and the pusher 18 from a top view (i.e., a superior view). As noted above, the cannula 14 is configured to penetrate soft tissue. To this end, the cannula 14 is tapered to a sharp, pointed distal end 44 in this example. The cannula 14 further includes a tubular bore 46, which receives the suture-implant construct 22 and the pusher 18. In this example, the cannula 14 further includes a slot 48 in a superior surface thereof. The slot 48 extends parallel to the longitudinal axis A of the cannula 14. The slot 48 includes opposing side walls 50, 52 which serve to guide movement of the pusher 18 in a direction parallel to the longitudinal axis A. The slot 48 may extend along a portion of the length of the cannula 14 or alternatively may extend along the entire length of the cannula 14.

The pusher 18 is configured to deploy a distal-most implant of the suture-implant construct 22 and is also configured to move any additional implants distally within the cannula. Specifically, in the example of the first and second implants 26A, 26B, distal movement of the pusher 18 is configured to deploy the first implant 26A and to move the second implant 26B distally within the cannula 14 to a deploy position. A second distal movement of the pusher 18 will then deploy the second implant 26B.

In one example, the pusher 18 includes a rod or shaft mechanically coupled to the trigger 16. The pusher 18 is configured to move in the distal and proximal directions in response to corresponding movement of the trigger 16. The pusher 18 further includes a blunt distal end 54 configured to push an implant in the distal direction. The distal end 54 in this example is a substantially planar surface arranged normal to the longitudinal axis A. The distal end 54 could be inclined at an acute angle relative to the longitudinal axis A. The pusher 18 further includes a relatively smooth superior surface 56, and relatively smooth side surfaces 58, 60 configured to slide relative to the respective side walls 50, 52 of the slot 48. The inferior surface 62 of the pusher 18 includes a shuttling rack 64 in this example, which is configured to interact with implants to move them distally within the cannula 14.

Figure 4 illustrates the shuttling rack 64 in greater detail. In this example, the shuttling rack 64 is integrated into the pusher 18, meaning the shuttling rack 64 and pusher 18 are a single, integrated structure. The shuttling rack 64 includes a plurality of barbs 66 projecting from the inferior surface 62 of the pusher 18. The barbs 66 each include a distal face 68 and a proximal face 70. The distal and proximal faces 68, 70 meet at an apex 72, which provides a relatively sharp point and is configured to engage an implant. In this example, the distal faces 68 are substantially normal to the distal direction and the longitudinal axis A, and the proximal faces 70 are inclined toward the proximal direction such that they project from the apex 72 at an acute angle 74 relative to the distal faces 68.

The shuttling rack 64 is configured to move implants distally when the pusher 18 moves distally, and is also configured to not move implants proximally as the pusher 18 moves proximally. Thus, during a sequence where the user U moves the trigger 16 in the distal direction and the trigger 16 moves back proximally under the bias of the spring, for example, the implants within the cannula will only move in the distal direction.

In this example, the pusher 18 does not include barbs 66 along the entirety of its length. Rather, as shown in Figure 5, there is a section of the pusher 18 adjacent the distal end 54 where the inferior surface 62 is smooth. The inferior surface 62 may be smooth along a length corresponding to the length of the implants 26A, 26B. Alternatively, the inferior surface 62 may have barbs 66 along its entire length.

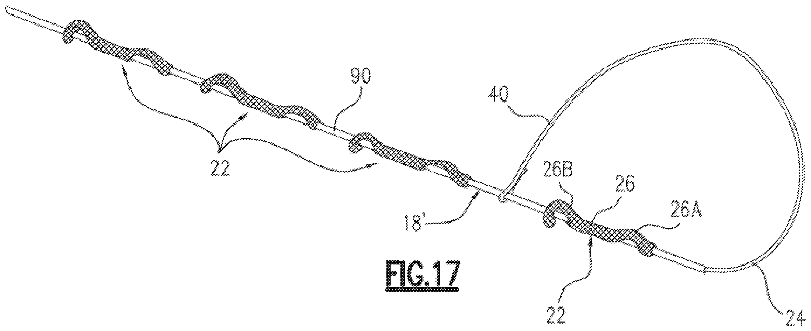

In an alternative embodiment, the pusher 18' of the surgical device may be a holder tube 90 which extends through the bore 34 of one or more of the sheaths 26 via the splice points of each sheath 26, as best seen in Figure 17. The sheaths 26 may be spaced apart from one another on the holder tube 90. The holder tube 90 and the sheaths 26 may reside in a cannula. The holder tube 90 is free to move back and forth relative to the sheaths 26. The sheaths 26 may be seated inside the cannula such that once the holder tube 90 moves behind the sheaths, the holder tube 90 will push the sheaths from behind. In this embodiment, the strand 24 may be extended or threaded through the holder tube 90 such that each sheath 26 may slide onto the strand 24 when the holder tube 90 is behind the sheaths, thereby deploying the first anchor portion 26A and any subsequent anchor portions of each sheath 26. The end 40 of the strand 24 may be affixed to the holder tube 90 in any known manner, such as a loop, a knot, or other fixation technique.

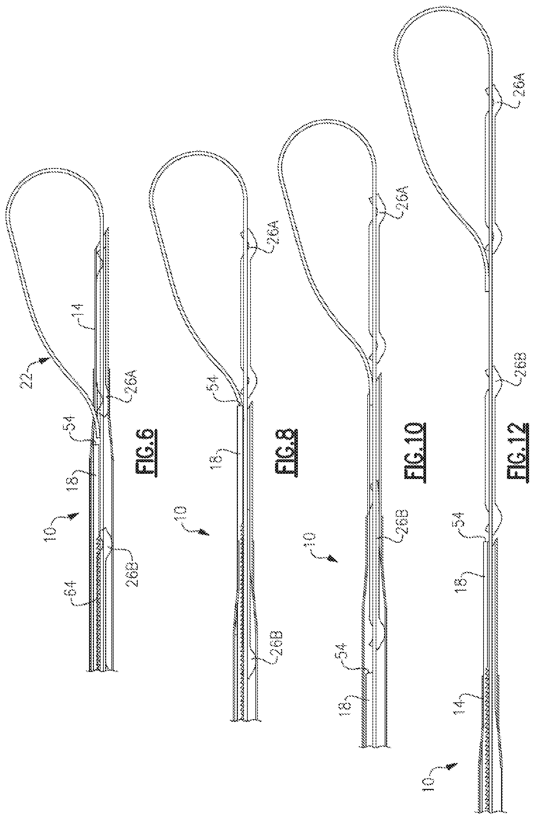

A method of using the surgical device 10 will now be described with reference to Figures 6-16. Figure 6 is a cross-sectional view of the surgical device 10 and illustrates the surgical device 10 with the suture-implant construct 22 loaded into the cannula 14. In particular, each of the first and second implants 26A, 26B are positioned within the cannula 14. The first implant 26A is a distal-most implant within the cannula 14. The distal end 54 of the pusher 18 is proximal to the first implant 26A, and the shuttling rack 64 is contact with the second implant 26B.

For purposes of this disclosure, the distal-most implant within the cannula 14 is in a "deploy position" in which the implant is ready to be deployed by the pusher 18, and any remaining implants are in a "standby position." The implants in the standby position are essentially waiting to be moved to the deploy position and ultimately deployed by the pusher 18. In Figure 6, the first implant 26A is in the deploy position and the second implant 26B is in a standby position.

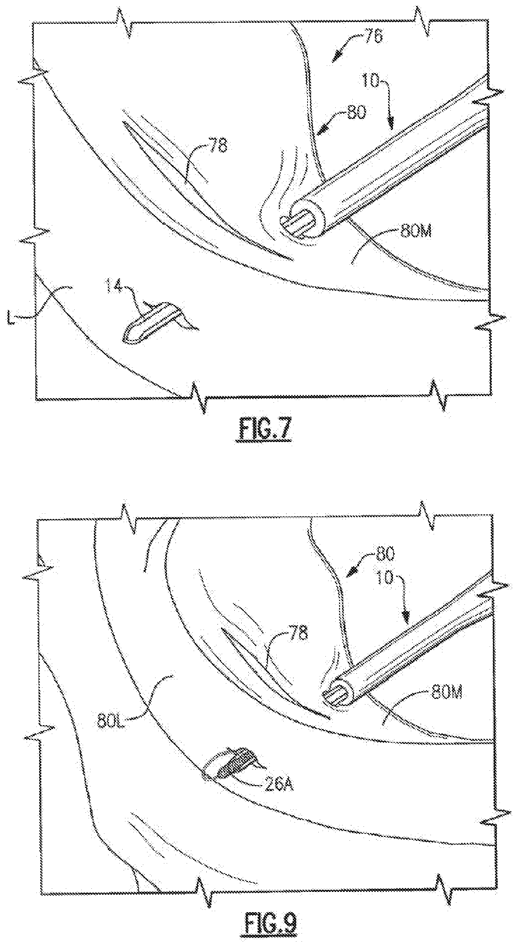

When in the position of Figure 6, a user U can navigate the surgical device 10 within a joint space 76, as illustrated in Figure 7. The joint space 76 in Figure 7 is a joint cavity in a knee, and is specifically a cavity between a femur and a tibia. While a knee joint is illustrated, it should be understood that this disclosure extends to other joints. As illustrated in Figure 7, there is a tear 78 in a meniscus 80. The surgical device 10 is used to deploy a plurality of implants in the area adjacent the tear 78 to close the tear 78 and allow it to heal.

In Figure 7, a user U penetrates the cannula 14 through the meniscus 80 in a first location where the first anchor portion 26A is to be deployed. In particular, the user penetrates the cannula 14 into the medial surface 80M and out the lateral surface 80L. Alternatively, the user could penetrate the cannula 14 into the lateral surface 80L and out the medial surface 80M. When the cannula 14 is in the first location, the user U applies an input force to the trigger 16, which moves the pusher 18 distally. In Figure 8, movement of the pusher 18 in the distal direction has deployed the first anchor portion 26A out of the cannula 14 and into a desired location adjacent the meniscus 80. Figure 9 illustrates the first anchor portion 26A as it is being deployed in the first location adjacent the lateral surface 80L of the meniscus. Further, as shown in Figure 8, the shuttling rack 64 has moved the second anchor portion 26B distally within the cannula relative to its position in Figure 6.

After the first implant 26A is deployed, the user U can remove their thumb, for example, from the trigger 16 allowing the trigger 16 and pusher 18 to move proximally to the position shown in Figure 10. Again, proximal movement of the pusher 18 does not move the second implant 26B proximally because of the arrangement of the shuttling rack 64, discussed above. In Figure 10, the second implant 26B is the distal-most implant in the cannula 14 and is distal to the distal end 54 of the pusher 18. Thus, the second implant 26B has moved from a standby position (e.g., Figures 6, 8) to the deploy position (e.g., Figure 10).

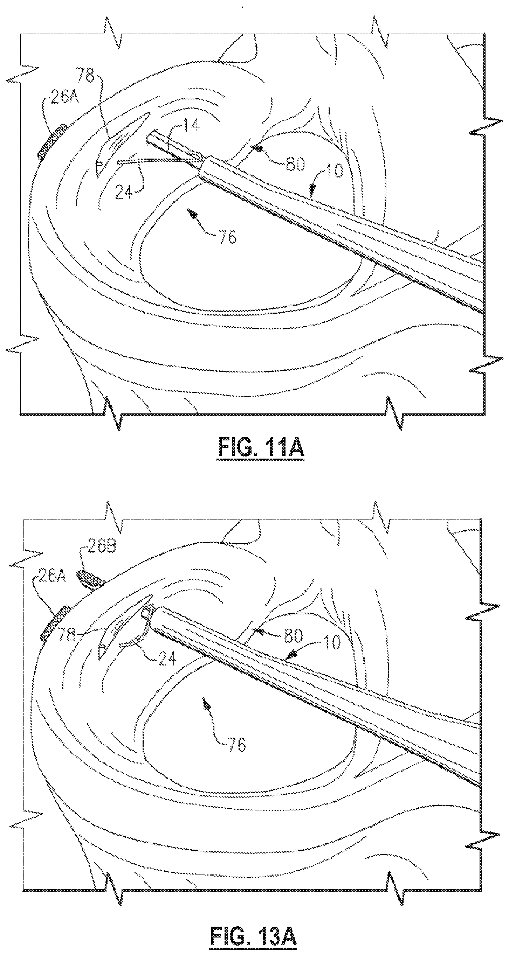

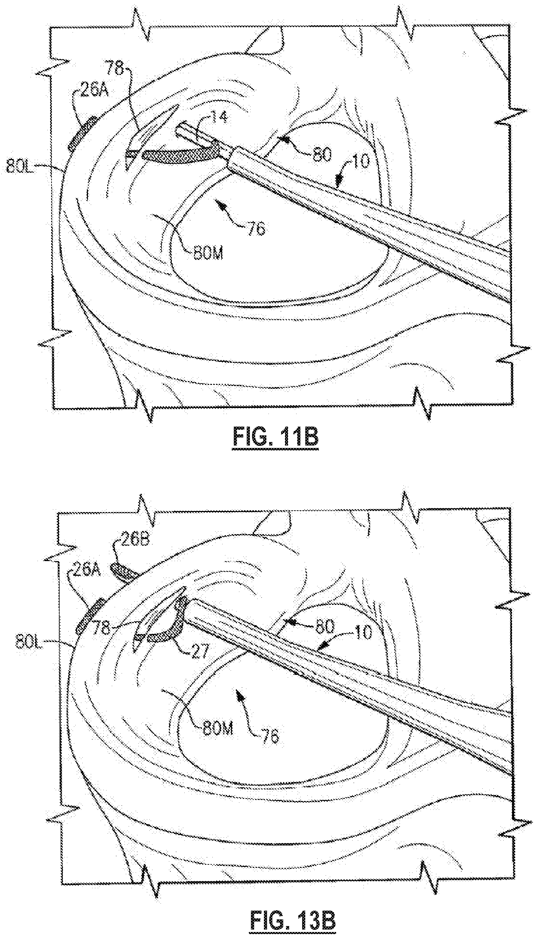

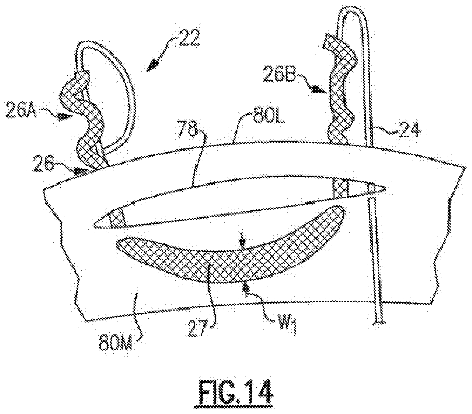

When in the position of Figure 10, the user U can position the cannula 14 in a second location adjacent the tear 78, as shown in Figures 11A and 11B. The second location is spaced-apart from the first location of Figure 7. Once in the second location, the user U can penetrate the meniscus 80 and move the trigger 16 in the distal direction, which moves the pusher 18 in the distal direction, and deploys the second implant or second anchor portion 26B, as illustrated in Figure 12. Figure 13A and 13B illustrate the second implant or the second anchor portion 26B as it is being deployed in the second location. Figure 14 illustrates the suture-implant construct 22 after the second anchor portion 26B has been deployed from a top, or superior view. As shown, the first and second anchor portions 26A, 26B are adjacent the lateral surface 80L of the meniscus 80, while the central portion 27 of the sheath spans between the first and second anchor portion 26A, 26B, including extending through the meniscus 80 and along the medial surface 80M adjacent the tear 78.

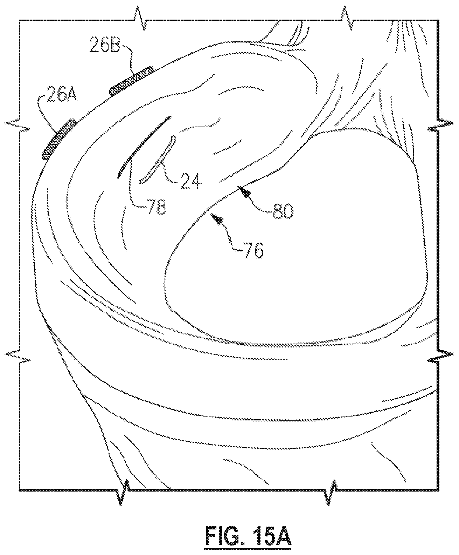

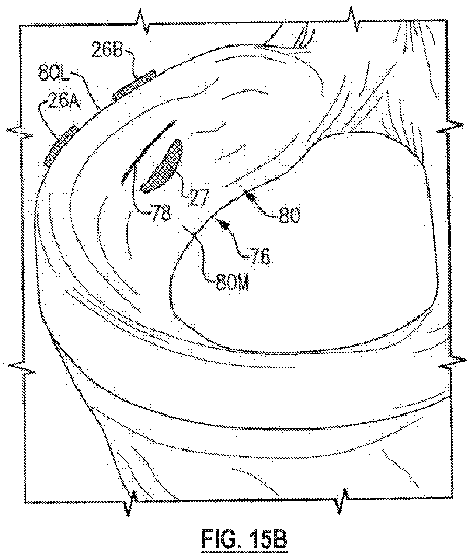

As seen in Figure 15A, once the first and second implants 26A, 26B are deployed, the suture 24 can then be tensioned, knotted, and trimmed to close the tear 78. As seen in Figure 15B, once the first and second implants or anchor portions 26A, 26B are deployed, the strand of suture 24 is tensioned. Doing so cinches or tensions the first and second anchor portions 26A, 26B such that the sheath 26 essentially bunches together and forms an anchor to close the tear 78. The tensioned strand of suture 24 applies a force to the meniscus 80. The increased width dimension W₁ of anchor portions 26A, 26B disperses that force over a relatively large area of the meniscus 80, which, as mentioned above, reduces the risk of "tear through."

While the suture-implant construct 22 may include two anchor portions 26A, 26B, additional anchor portions can be used to repair larger tears. In that case, the trigger 16 can be activated additional times to deploy each additional implant.

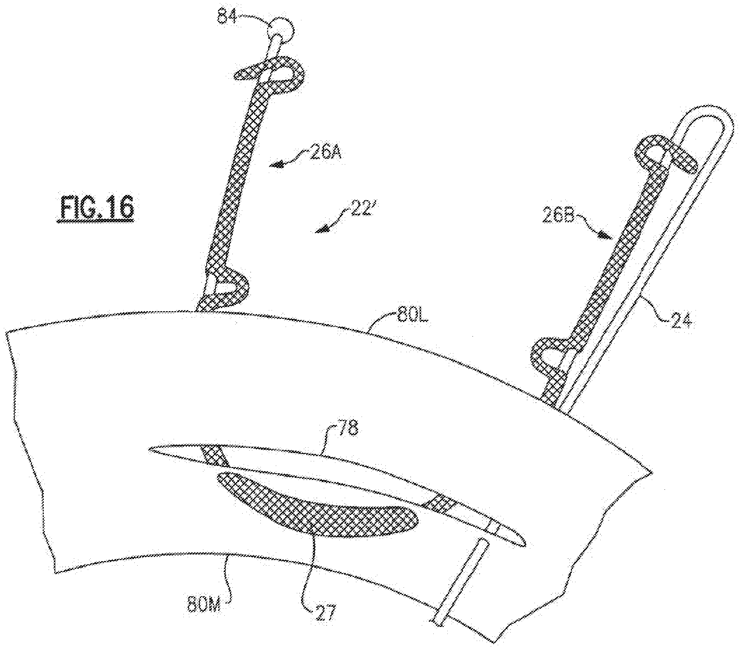

While in Figures 2A and 2B the strand of suture 24 is retained relative to the sheath 26 by being looped-back and affixed to itself, the strand of suture 24 could be retained in other ways. Figure 16, for example, illustrates a suture-implant construct 22' arranged relative to a meniscus 80 in substantially the same way as Figure 14. The strand of suture 24, in this embodiment, includes a bulb 84 at a distal end thereof. The bulb 84 has a diameter larger than the bore of the sheath 26, which prevents the sheath 26 from sliding distally off the strand of suture 24. While a loop (e.g., Figures 2A and 2B) and bulb (e.g., Figure 16) have been shown, this disclosure extends to other techniques for retaining the strand of suture 24 relative to the sheath 26.

It should be understood that terms such as "distal," "proximal," "superior," and "inferior" are used above consistent with the way those terms are used in the art. Further, these terms have been used herein for purposes of explanation, and should not be considered otherwise limiting. Terms such as "generally," "substantially," and "about" are not intended to be boundaryless terms, and should be interpreted consistent with the way one skilled in the art would interpret those terms.

Although the different examples have the specific components shown in the illustrations, embodiments of this disclosure are not limited to those particular combinations. It is possible to use some of the components or features from one of the examples in combination with features or components from another one of the examples.

## Claims

1. A surgical device (10), comprising:
a cannula (14);
a suture-implant construct (22) comprising a strand of suture (24) and at least two implants (26A, 26B), wherein a first implant (26A) is in a deploy position, and a second implant (26B) is in a standby position proximal to the first implant (26A); and
a pusher (18) moveable within the cannula (14) to deploy the first implant (26A) and to move the second implant (26B) from the standby position to the deploy position,
wherein the pusher (18) includes a shuttling rack (64) in contact with the second implant (26B) when the second implant (26B) is in the standby position, and
wherein the pusher (18) includes an elongate shaft extending along the cannula (14)
**characterized in that**
the shuttling rack (64) includes a plurality of barbs (66) on a side of the elongate shaft.

2. The surgical device (10) as recited in claim 1, the shuttling rack (64) is integrated into the pusher (18).

3. The surgical device (10) as recited in claim 1, wherein the cannula (14) includes a slot, and wherein the pusher (18) is arranged within the slot.

4. The surgical device (10) as recited in claim 1, wherein each of the plurality of barbs (66) has a distal face (68) and proximal face (70) meeting at an apex, and wherein the distal face (68) is substantially normal to a longitudinal axis of the cannula (14), and the proximal face (70) is inclined at an acute angle relative to the distal faces (68).

5. The surgical device (10) as recited in claim 1, further comprising a trigger (16) configured to move in a distal direction and a proximal direction, wherein the trigger (16) is mechanically coupled to the pusher (18) such that movement of the trigger (16) moves the pusher (18).

6. The surgical device (10) as recited in claim 5, wherein the trigger (16) is a thumb trigger.

7. The surgical device (10) as recited in claim 5, wherein the trigger (16) is biased in the proximal direction.

8. The surgical device (10) as recited in claim 5, further comprising a brake configured to be selectively activated to overcome the bias of the trigger (16) and maintain a position of the trigger (16) and the pusher (18).

9. The surgical device (10) as recited in claim 1, wherein the first and second implants (26A, 26B) are tubular sleeves made of a flexible material.

10. The surgical device (10) as recited in claim 9, wherein the first and second implants (26A, 26B) are made of a polyester suture material.

11. The surgical device (10) as recited in claim 1, wherein the suture-implant construct (22) includes a third implant proximal to the second implant and a fourth implant proximal to the third implant.

## Patentansprüche

1. Chirurgische Vorrichtung (10), Folgendes umfassend: eine Kanüle (14);
ein Naht-Implantat-Konstrukt (22), umfassend einen Nahtfadenstrang (24) und mindestens zwei Implantate (26A, 26B), wobei ein erstes Implantat (26A) sich in einer Bereitstellungsposition befindet und ein zweites Implantat (26B) sich in einer Bereitschaftsposition proximal zum ersten Implantat (26A) befindet; und
einen Schieber (18), der innerhalb der Kanüle (14) beweglich ist, um das erste Implantat (26A) bereitzustellen und um das zweite Implantat (26B) von der Bereitschaftsposition in die Bereitstellungsposition zu bewegen;
wobei der Schieber (18) ein sich hin- und herbewegendes Gestell (64) in Kontakt mit dem zweiten Implantat (26B) beinhaltet, wenn sich das zweite Implantat (26B) in der Bereitschaftsposition befindet; und
wobei der Schieber (18) einen länglichen Schaft beinhaltet, der sich entlang der Kanüle (14) erstreckt, **dadurch gekennzeichnet, dass**
das hin- und herbewegende Gestell (64) auf einer Seite des länglichen Schafts eine Vielzahl von Widerhaken (66) beinhaltet.

2. Chirurgische Vorrichtung (10) nach Anspruch 1, wobei das sich hin- und herbewegende Gestell (64) in den Schieber (18) integriert ist.

3. Chirurgische Vorrichtung (10) nach Anspruch 1, wobei die Kanüle (14) einen Schlitz aufweist, und wobei der Schieber (18) innerhalb des Schlitzes angeordnet ist.

4. Chirurgische Vorrichtung (10) nach Anspruch 1, wobei jeder der Vielzahl von Widerhaken (66) eine distale Fläche (68) und eine proximale Fläche (70) aufweist, die an einem Scheitel zusammentreffen, und wobei die distale Fläche (68) im Wesentlichen senkrecht zu einer Längsachse der Kanüle (14) ist und die proximale Fläche (70) in einem spitzen Winkel relativ zu den distalen Flächen (68) geneigt ist.

5. Chirurgische Vorrichtung (10) nach Anspruch 1, ferner umfassend einen Auslöser (16), der so konfiguriert ist, dass er sich in einer distalen Richtung und einer proximalen Richtung bewegt, wobei der Auslöser (16) mechanisch mit dem Schieber (18) gekoppelt ist, sodass eine Bewegung des Auslösers (16) den Schieber (18) bewegt.

6. Chirurgische Vorrichtung (10) nach Anspruch 5, wobei der Auslöser (16) ein Daumen-Auslöser ist.

7. Chirurgische Vorrichtung (10) nach Anspruch 5, wobei der Auslöser (16) in der proximalen Richtung vorgespannt ist.

8. Chirurgische Vorrichtung (10) nach Anspruch 5, ferner umfassend eine Bremse, die so konfiguriert ist, dass sie selektiv aktiviert wird, um die Vorspannung des Auslösers (16) zu überwinden und eine Position des Auslösers (16) und des Schiebers (18) beizubehalten.

9. Chirurgische Vorrichtung (10) nach Anspruch 1, wobei das erste und das zweite Implantat (26A, 26B) röhrenförmige Hülsen aus einem biegsamen Material sind.

10. Chirurgische Vorrichtung (10) nach Anspruch 9, wobei das erste und das zweite Implantat (26A, 26B) aus einem Polyester-Nahtmaterial hergestellt sind.

11. Chirurgische Vorrichtung (10) nach Anspruch 1, wobei das Naht-Implantat-Konstrukt (22) ein drittes Implantat proximal zum zweiten Implantat und ein viertes Implantat proximal zum dritten Implantat beinhalt.

## Revendications

1. Dispositif chirurgical (10), comprenant :
une canule (14) ;
une structure suture-implant (22) comprenant un brin de suture (24) et au moins deux implants (26A, 26B), dans lequel un premier implant (26A) est dans une position de déploiement, et un deuxième implant (26B) est dans une position d'attente à proximité du premier implant (26A) ; et
un poussoir (18) déplaçable à l'intérieur de la canule (14) pour déployer le premier implant (26A) et pour déplacer le deuxième implant (26B) de la position d'attente à la position de déploiement,
dans lequel le poussoir (18) inclut une crémaillère de navette (64) en contact avec le deuxième implant (26B) lorsque le deuxième implant (26B) est dans la position d'attente, et
dans lequel le poussoir (18) inclut une tige allongée s'étendant le long de la canule (14) **caractérisé en ce que**
la crémaillère de navette (64) comprend une pluralité de barbillons (66) sur un côté de la tige allongée.

2. Dispositif chirurgical (10) selon la revendication 1, dans lequel la crémaillère de navette (64) est intégrée dans le poussoir (18).

3. Dispositif chirurgical (10) selon la revendication 1, dans lequel la canule (14) inclut une fente, et dans lequel le poussoir (18) est disposé à l'intérieur de la fente.

4. Dispositif chirurgical (10) selon la revendication 1, dans lequel chacun de la pluralité de barbillons (66) a une face distale (68) et une face proximale (70) se rencontrant au niveau d'un apex, et dans lequel la face distale (68) est sensiblement normale à un axe longitudinal de la canule (14), et la face proximale (70) est inclinée à un angle aigu par rapport aux faces distales (68).

5. Dispositif chirurgical (10) selon la revendication 1, comprenant en outre un déclencheur (16) configuré pour se déplacer dans une direction distale et une direction proximale, dans lequel le déclencheur (16) est couplé mécaniquement au poussoir (18) de telle sorte que le déplacement du déclencheur (16) déplace le poussoir (18).

6. Dispositif chirurgical (10) selon la revendication 5, dans lequel le déclencheur (16) est un déclencheur actionné par le pouce.

7. Dispositif chirurgical (10) selon la revendication 5, dans lequel le déclencheur (16) est sollicité dans la direction proximale.

8. Dispositif chirurgical (10) selon la revendication 5, comprenant en outre un frein configuré pour être sélectivement activé pour surmonter la sollicitation du déclencheur (16) et maintenir une position du déclencheur (16) et du poussoir (18).

9. Dispositif chirurgical (10) selon la revendication 1, dans lequel les premier et deuxième implants (26A, 26B) sont des manchons tubulaires composés d'un matériau flexible.

10. Dispositif chirurgical (10) selon la revendication 9, dans lequel les premier et deuxième implants (26A, 26B) sont composés d'un matériau de suture en polyester.

11. Dispositif chirurgical (10) selon la revendication 1, dans lequel la structure suture-implant (22) inclut un troisième implant proximal au deuxième implant et un quatrième implant proximal au troisième implant.
